# EUROPÄISCHE PATENTSCHRIFT

(11) **EP 1 876 948 B1**
(45) Veröffentlichungstag und Bekanntmachung des Hinweises auf die Patenterteilung: **13.01.2010**
(21) Anmeldenummer: 06753446.1
(22) Anmeldetag: 28.04.2006
(51) Int. Cl.: A61B 5/0215, A61M 25/06

(54) **VORRICHTUNG ZUR ERFASSUNG PHYSIOLOGISCHER MESSGRÖSSEN IM KÖRPERINNEREN**
DEVICE FOR DETECTING PHYSIOLOGICAL MEASURED VARIABLES WITHIN THE BODY
DISPOSITIF PERMETTANT L'ACQUISITION DE GRANDEURS DE MESURE PHYSIOLOGIQUES A L'INTERIEUR D'UN CORPS

(30) Priorität: 03.05.2005 DE 102005020980; 27.07.2005 DE 102005035795
(43) Veröffentlichungstag der Anmeldung: 16.01.2008
(73) Patentinhaber: MHM Harzbecher Medizintechnik GmbH, 63739 Aschaffenburg (DE)
(72) Erfinder: RUHL, Karl, Michael, 52152 Simmerath (DE); PFEFFER, Joachim, Georg, 52070 Aachen (DE); SCHMITZ-RODE, Thomas, 52070 Aachen (DE); SCHNAKENBERG, Uwe, 52074 Aachen (DE)
(74) Vertreter: Borkowski, Jens
(86) Internationale Anmeldenummer: PCT/EP2006/004001
(87) Internationale Veröffentlichungsnummer: WO 2006/117154

(56) Entgegenhaltungen:
- WO-A-01/49363
- WO-A-2005/120336
- DE-U1- 8 204 827
- US-A1- 2004 167 580
- US-B1- 6 264 611
- HIEROLD C ET AL: "Implantable low power integrated pressure sensor system for minimal invasive telemetric patient monitoring" MICRO ELECTRO MECHANICAL SYSTEMS, 1998. MEMS 98. PROCEEDINGS., THE ELEVENTH ANNUAL INTERNATIONAL WORKSHOP ON HEIDELBERG, GERMANY 25-29 JAN. 1998, NEW YORK, NY, USA,IEEE, US, 25. Januar 1998 (1998-01-25), Seiten 568-573, XP010270264 ISBN: 0-7803-4412-X

## Beschreibung

Die Erfindung betrifft eine Vorrichtung zur Erfassung physiologischer Meßgrößen im Körperinneren umfassend einen Katheter mit einer sensortragenden Spitze, der in ein Blutgefäß einführbar ist.

Im Stand der Technik, z.B. aus der EP 0 646 350 A1 ist es bekannt, mittels eines Katheters eine Punktion eines Blutgefäßes durchzuführen, wobei über die Katheterspitze die Messung des Druckes im Lumen des Blutgefäßes möglich ist. Hierzu ist die Katheterspitze mit einem Druckmessinstrument verbunden, welches bei der Katheterisierung außerhalb des Körpers eines behandelten Patienten liegt. Dieses Vorgehen entspricht einer invasiven Untersuchungsmaßnahme, bei der der aus dem Körper herausragende Katheter eine Eintrittspforte für Keime darstellt. Weiterhin sind Katheter bekannt, die an ihrer Spitze einen Sensor aufweisen, um physiologische Meßgrößen, insbesondere intravaskuläre Meßgrößen aufzunehmen.

Aus der US 2004/167580 A1 ist eine Vorrichtung zur Erfassung physiologischer Messgrössen im Körperinneren umfassend einen Katheter mit einer sensortragenden Spitze, der in ein Blutgefäss einführbar ist, bekannt bei der der Katheter mit einer Transpondereinheit zur drahtlosen Übertragung wenigstens einer Messgrösse an eine äussere Lesestation verbunden ist, die nach Einführung des Katheters in ein Blutgefäss ausserhalb des Blutgefässes angeordnet ist, wobei Katheter und Transponder flüssigkeitsdicht versiegelt sind.

Die WO 01/49363 A1 zeigt ein auftrennbares medizinisches Ventil, mit einem Durchgang zur Aufnahme eines Katheters in dem ein Dichtungselement angeordnet ist und das geeignet ist am distalen Ende eine aufreißbare Hülse aufzunehmen.

Grundsätzlich problematisch ist es, dass ein Katheter durch eine Gefäßwandpunktion in das Gefäßlumen eingebracht werden muss. Insbesondere bei Punktion einer Arterie (Schlagader) sollte das Wandtrauma und damit eine Blutung minimiert werden. Hierbei ist es bekannt, zur Einführung eines Katheters in ein Blutgefäß Schleusen zu verwenden sowie auch blutstillende Elemente.

Selbst bei Entfernung einer sehr dünnen Schleuse zur Kathetereinführung in das Gefäß kann eine Blutung nicht vermieden werden. Bei einem ambulant implantierbaren Katheter ist aber eine schnelle und dauerhafte Blutstillung ganz wichtig, weil das Punktionsloch in der Gefäßwand immer etwas größer ist als der anschliessend eingelegte Katheter.

Problematisch sind Thrombenablagerungen am Katheter. Sie müssen vermieden werden, da sie zu Embolien im Gefäßgebiet stromab führen und bei Überdeckung der Sensorfläche zu Fehlmessungen führen und schlimmstenfalls das gesamte Blutgefäß verstopfen können.

Es muss weiterhin für eine ausreichende Implantationstiefe des Katheters im Blutgefäß gesorgt werden und es muss für den Untersucher diesbezüglich eine Orientierung geschaffen werden. Eine Dislokation des Katheters, z.B. durch Kräfte und Relativbewegungen am nicht im Gefäß befindlichen Katheterstück bedingt durch Muskel- und Gelenkbewegungen, muss verhindert werden. Schlimmstenfalls würde ein Herausrutschen des Katheters aus dem Blutgefäß eine eventuell lebensbedrohliche Blutung zur Folge haben.

Der Sensor muss so platziert sein, dass im Falle einer Druckmessung der rein statische Druck erfasst wird. Die additive Messung eines Staudruckanteils muss vermieden werden.

Die bekannten Katheter haben weiterhin den Nachteil, dass das Katheterende außerhalb des Körpers liegt und so eine Keim-Eintrittspforte darstellen, mit der eine Langzeitüberwachung nicht möglich ist. Insbesondere kann ein Patient eine solche Katheteranordnung nicht dauerhaft mit sich führen.

Aufgabe ist es, die vorgenannten Nachteile des Standes der Technik zu überwinden und insbesondere ein Katheter bzw. ein Kathetersystem zu schaffen, mittels dem bei Punktion einer Arterie (Schlagader) das Wandtrauma minimierbar und eine Blutung verhinderbar ist. Weiterhin soll eine schnelle und dauerhafte Blutstillung herbeizuführen sein, auch wenn das Punktionsloch in der Gefäßwand etwas größer ist als der einzuführende bzw. zu implantierende Katheter. Schleusen zur Kathetereinführung in das Gefäß sollen ablösbar gestaltet sein, ohne dass Kabel- oder Katheterverbindungen gelöst werden müssen.

Gemäß weiterer Aufgaben sollen Thrombenablagerungen am Katheter vermieden werden, eine ausreichende Implantationstiefe des Katheters im Blutgefäß erreicht werden und für den Untersucher diesbezüglich eine Orientierung geschaffen werden. Es gilt eine Dislokation des Katheters zu verhindern und den Sensor so zu platzieren, dass im Falle einer Druckmessung der rein statische Druck erfasst wird und die additive Messung eines Staudruckanteils vermieden wird.

Erfindungsgemäß werden die Aufgabe durch die Vorrichtung gemäß Anspruch 1 gelöst.

Dadurch, dass Katheter und Transponder flüssigkeitsdicht versiegelt sind und eine Einheit bilden, kann diese Anordnung auch für Langzeituntersuchungen im Körper eines Patienten verbleiben. Mittels des Sensors in der Spitze des Katheters können beliebige Meßgrößen erfasst und mittels des Transponders an eine außerhalb des Körpers liegende Leseeinheit übermittelt werden. Hierfür kann z.B. die übliche RFID-Technik zum Einsatz kommen.

Beispielhaft und ohne Beschränkung der Erfindung wird die Anwendung bei einem Blutdruckmesssystem gemäß den nachfolgenden Figuren beschrieben. Es zeigen:
- Figur 1:: eine Schleuse mit und ohne Punktionsnadel;
- Figur 2:: ein erfindungsgemäßer Katheter nach Einführung in ein Blutgefäß mit den den Katheter koaxial umgebenden Hilfselementen;

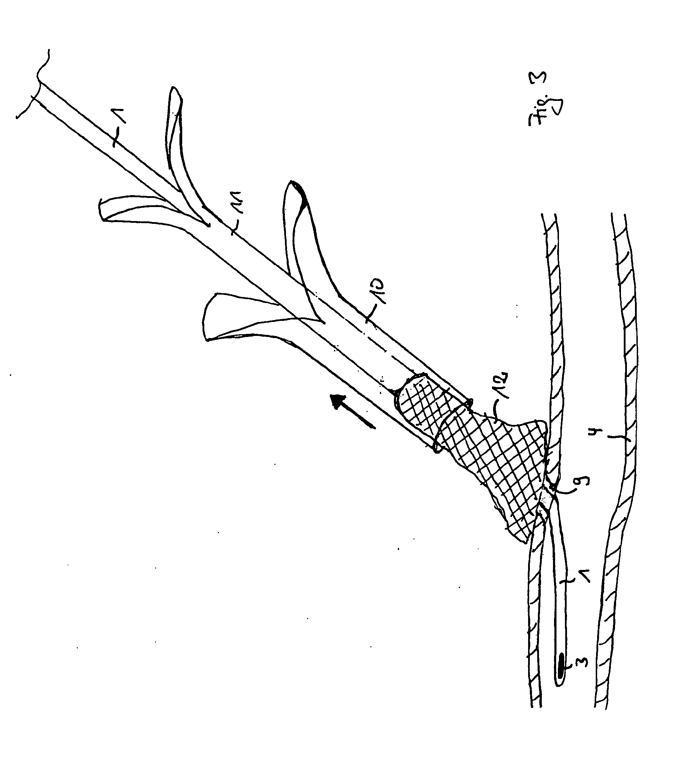

- Figur 3:: das gefäßwandnahe Platzieren einer blutstillenden Manschette mittels der Hilfselemente;
- Figur 4:: eine Gesamtübersicht mit Positionsmarkierungen am Katheter;
- Figur 5:: eine Gesamtübersicht mit Fixationsclip am Katheter;
- Figur 6:: eine Gesamtübersicht mit einem Kanal im Katheter als Positionierungshilfe;

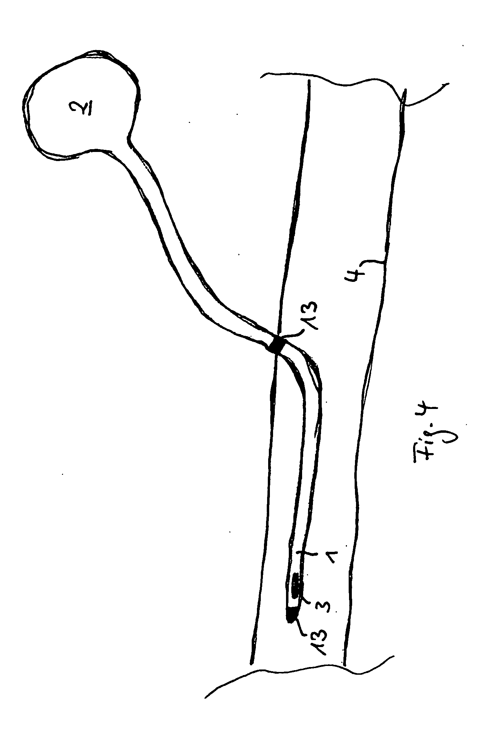

Das telemetrische Blutdruckmesssystem, welches in den Figuren 4, 5 und 6 in einer Gesamtübersicht und unterschiedlichen, auch kombinierbaren Ausführungen dargestellt ist, ist komplett vormontiert und flüssigkeitsdicht verkapselt. Der Sensor-Katheter 1 und die Transpondereinheit 2 sind dabei miteinander ohne lösbare Verbindung verkabelt.

Hierdurch wird eine verbesserte Handhabbarkeit, Bioverträglichkeit und Dauerhaftigkeit des Implantats bewirkt. Der Katheter 1 wird mit seiner sensortragenden Spitze 3 in ein Blutgefäß 4 eingeführt, d.h. implantiert, wobei die in Verbindung stehende Transpondereinheit 2 bevorzugt im umliegenden Gewebe implantiert wird, d.h. außerhalb des Blutgefäßes 4, z.B. im subkutanen Fettgewebe. Mittels einer hier nicht dargestellten Leseeinheit können Meßgrößen aus dem Blutgefäß drahtlos aus der Transpondereinheit 2 ausgelesen werden.

Bei einem üblichen Punktionsvorgang eines Blutgefäßes 4 wird mit einer Stahlnadel 5 das Gefäß 4 punktiert und dabei eine auf der Stahlnadel 5 befindliche Schleuse 6 in das Gefäß 4 eingebracht. Dann wird die Nadel 5 entfernt und der Katheter 1 durch die liegende Schleuse 6 eingeführt. Nach Kathetereinlage muß die Schleuse 6 entfernt werden. Normalerweise werden Schleusen zur Entfernung koaxial vom Katheter 1 abgestreift. Die Schleuse ist eine im wesentlichen schlauchartige Hülse, die koaxial die Nadel oder andere Elemente umgibt, die in eine Blutgefäß eingeführt werden sollen. Im weiteren werden die Begriffe Schleuse/Hülse synonym verwendet.

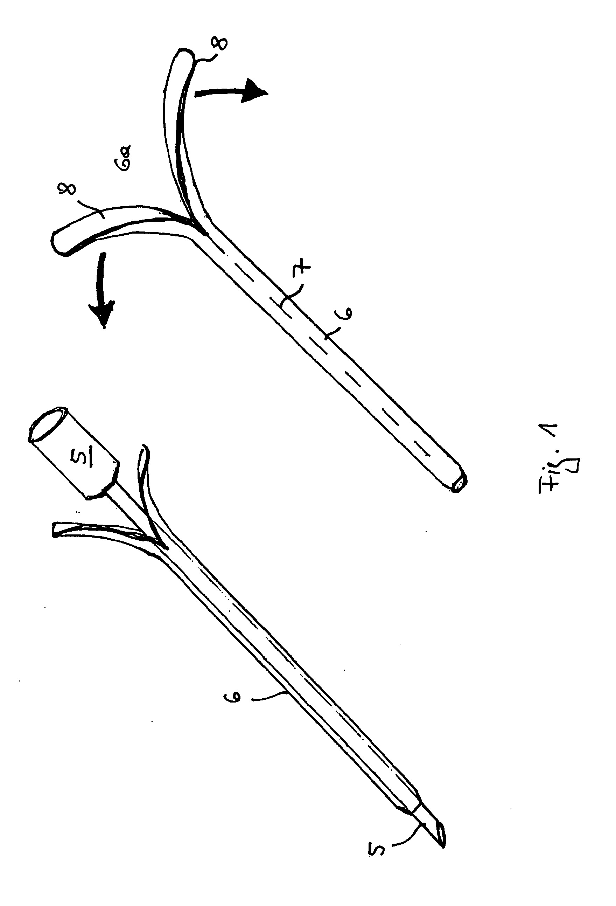

Bei dem hier zum Einsatz kommenden vormontierten System nach Figuren 4 bis 6 ist die koaxiale Entfernung der Schleuse nicht möglich, da die Transpondereinheit 2 fest am Katheter 1 angeschlossen ist. Die Implantation des Sensor-Katheters 1 erfolgt daher durch eine Schleuse 6, die in üblicher Weise gemäß Figur 1 mittels der vorgenannten Nadel 5 gesetzt werden kann und die in Längsrichtung wenigstens einmal teilbar ist, bevorzugt zweifach.

Hierfür weist sie wenigstens über einen Teil ihrer Länge, bevorzugt über die volle Länge, wenigstens eine, bevorzugt an ihrem äußeren Ende 6a beginnende, insbesondere zwei gegenüberliegende, in Längsrichtung verlaufende Sollriss-Stellen, z.B. Schlitze 7 auf. So kann nach Katheterplatzierung und Rückzug aus dem Gefäß die Hülse 6 an wenigstens einer Sollriss-Stelle 7 aufgetrennt und vom Katheter 1 abgeschält werden, bevorzugt in zwei Hälften 8. Damit die komplette Abschälbarkeit (Teilbarkeit auf ganzer Länge in zwei Hälften) gegeben ist, kann bevorzugt als Material für eine solche Schleuse PTFE verwendet werden.

Nach Punktion des Blutgefäßes 4 mit einer Stahlnadel 5, Einbringung der Hülse 6 in das Gefäß 4, Entfernung der Stahlnadel 5, Einführung des Katheters 1 mit der sensortragenden Spitze 3 einige Zentimeter in das Gefäß 4 und Rückzug sowie Abschälen der Schleuse aus dem Blutgefäß 4, entsteht eine Blutung, insbesondere aus einem arteriellen Hochdruckgefäß, da das Punktionsloch 9 in der Gefäßwand dem Außendurchmesser der entfernten Schleuse 6 entspricht, jetzt aber nur durch den einliegenden Katheter 1 mit einem kleineren Außendurchmesser gefüllt ist.

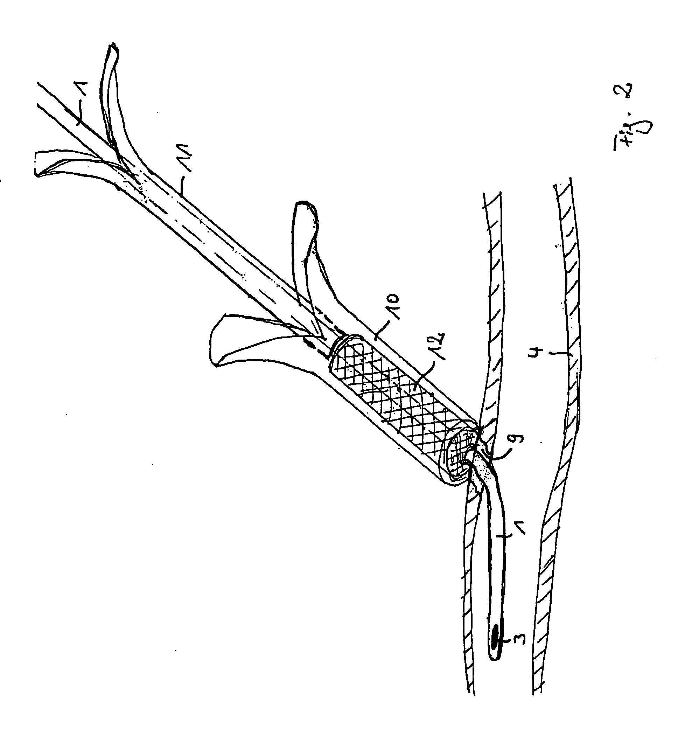

Erfindungsgemäß ist es vorgesehen sein, dass die Blutung z.B. durch Vorschieben einer koaxial auf dem Katheter 1 angeordneten, vormontierten weiteren Hülse 10 bis vor die äußere Gefäßwand gestoppt wird. Dies ist in Figur 2 erkennbar.

Eine nochmals weitere Hülse 11 dient als Haltehülse. Sie hat einen kleineren Durchmesser als die Hülse 10.

Innerhalb der Hülse 10, an deren dem Sensor zugewandten Ende, ist eine eingesetzte, insbesondere eingepresste z.B. hülsenförmige Manschette 12 z.B. aus blutstillendem Material (Hämostyptikum, z.B. Gelatine oder Kollagen) angeordnet. Bei der Manschette 12 kann es sich um einen gepressten Gelatineschwamm handeln.

Mittels der Haltehülse 11 kann die Manschette 12 in Position gehalten werden, während Hülse 10 gegen die fixierte Haltehülse 11 zurückbewegt wird und damit die Manschette 12 freigibt.

Durch Freigabe der Manschette 12 quillt diese vor der Gefäßwand auf. Dadurch wird die Blutung dauerhaft gestillt. Hülse 10 kann dann ebenfalls im Abschälverfahren z.B. in zwei Teile gespalten und entfernt werden. Hülse 10 kann hinsichtlich der Teilbarkeit, insbesondere in zwei Hälften dieselben Merkmale aufweisen, wie die Hülse 6 zur Einführung des Katheters 1.

Die ebenso in gleicher Weise abschälbare Hülse bzw. Schleuse 11, die auch aus PTFE bestehen kann, wird bevorzugt zum Festhalten der Manschette 12 verwendet, um diese freizusetzen, und besonders bevorzugt zusätzlich als Notschleuse, damit der Gefäßzugang erhalten bleibt, falls das Kathetersystem im Verlauf der Prozedur wieder entfernt werden muss.

Gemäß eines alternativen Beispiels kann auch eine fest auf dem Katheterschaft aufgebrachte Manschette aus bevorzugt quellbarem, insbesondere blutstillendem Material so durch Vorschieben des Katheters positioniert werden, dass sie sich genau in Höhe der Eintrittsstelle des Katheters 1 in die Gefäßwand befindet, und damit die Blutung stoppt. Der Nachteil gegenüber der zuvor beschriebenen Lösung ist jedoch, dass zumindest ein kleiner Teil dieser Manschette dann in Kontakt zum Blutstrom im Gefäßinneren gelangen kann und aufgrund der Thrombogenität des blutstillenden Materials zu Appositionsthrombenbildung und Mikroembolien führen kann.

Sollte die Katheterimplantation nicht reibungslos verlaufen sein oder das Implantat defekt sein, kann die vormontierte Hülse 11 als Notschleuse dienen, die wieder in das Gefäß 4 vorgeschoben werden kann und so den Gefäßzugang erhält. Die gesamte Prozedur kann so gegebenenfalls wiederholt werden. Auch die Hülse 11 kann hinsichlich der Abschälbarkeit dieselben Merkmale aufweisen wie zur Hülse 6 und Hülse 10 beschrieben.

Um sicherzustellen, dass der Katheter 1 unter Röntgen-Durchleuchtungs-kontrolle weit genug in das Gefäß 4 vorgeschoben wird, kann ein röntgendichter Markerring 13 an der Spitze 3 und einige Zentimeter von der sensortragenden Spitze 3 entfernt am Katheterschaft angebracht werden. Dies zeigt Figur 4. Der Katheter 1 kann so mit dem zweiten Markerring 13 in der Nähe des Gefäßeintritts des Katheters positioniert werden.

Alternativ oder additiv kann als zusätzliche Kontrolle, auch für die Positionierung unter Ultraschallkontrolle, ein Knick mit einem Winkel von 30 bis 90° im Katheterschaft dienen. Der Scheitelpunkt dieses Knicks kann in der Nähe des Gefäßeintritts des Katheters positioniert werden.

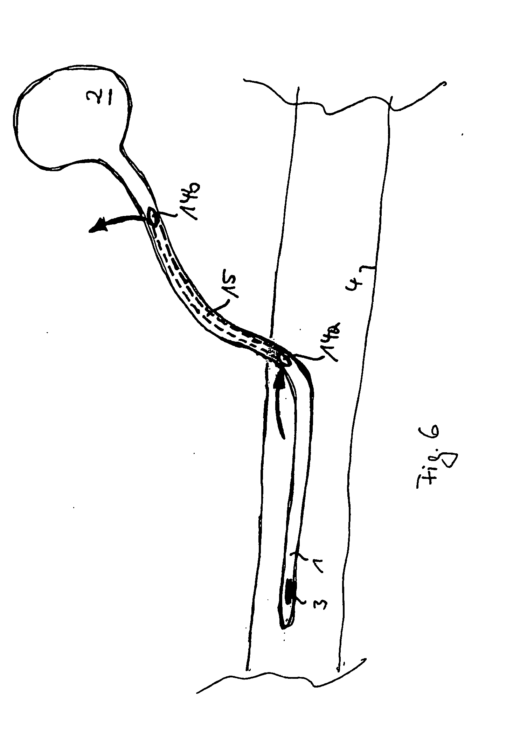

Als weitere Alternative zur Sicherstellung einer korrekten Katheterpositionierung kann auch ein kleines Seitloch 14a im Katheterschaft dienen, einige Zentimeter von der sensortragenden Spitze entfernt. Dieses Seitloch 14a ist über einen im Katheterinneren verlaufenden Kanal 15 mit einer Seitöffnung 14b im nicht für die Gefäßimplantation bestimmten, transpondernahen Abschnitt des Katheters 1 verbunden. Dies zeigt Figur 6. Bei Exposition des spitzennahen Seitlochs 14a mit dem Blutstrom im Gefäßinneren kommt es zu einem kontrollierten Blutrückstrom aus dem äußeren Seitloch 14b, aus der auf die Länge des im Gefäß befindlichen Abschnitts des Katheters 1 geschlossen werden kann. Um diesen Blutaustritt zu stoppen, kann der Katheter geringfügig zurückgezogen werden, so dass das spitzennahe Seitloch 14 a gerade nicht mehr im Gefäßlumen liegt.

Das zuvor beschriebene Seitloch-Kanalsystem 14/15 kann auch zur Injektion von Röntgen- oder Ultraschall-Kontrastmittel oder anderer Fluide in das Blutgefäß 4 genutzt werden, z.B. um das Blutgefäß während der Implantationsprozedur bildgebend besser darzustellen.

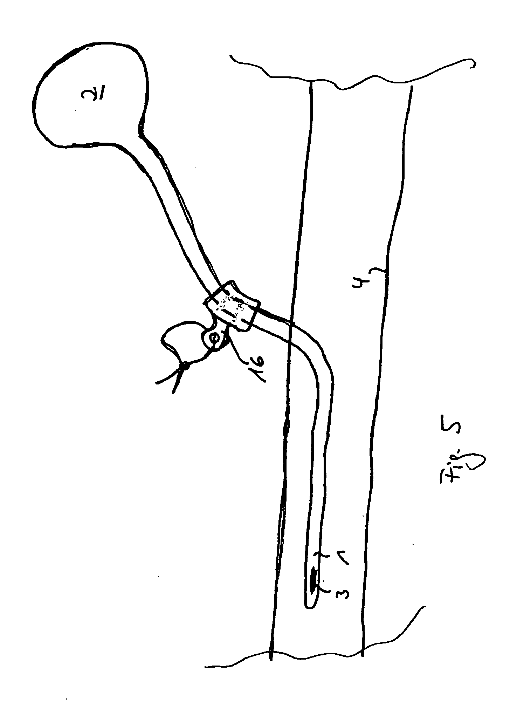

Um den Katheter langfristig in einer optimalen Position zu fixieren und ein sukzessives Herausrutschen aus dem Gefäß 4 z.B. durch Kräfte, die am außerhalb des Gefäßes 4 liegenden Katheterabschnitt wirken, zu verhindern, kann ein bevorzugt vorgesehener Fixationsclip 16 dienen, der einerseits den Katheterschaft umgreift und andererseits ein Befestigungselement, z.B. eine Öse o. ä. für z.B. eine Nahtfixation oder Klammerfixation am benachbarten Gewebe (z.B. Muskelfaszie) aufweist. Figur 5 zeigt diese Ausführung, die mit allen anderen Ausführungen kombinierbar ist. Sinnvollerweise befindet sich der Fixationsclip etwa 1 Zentimeter nach außen von der Gefäßeintrittsstelle des Katheterschaftes entfernt, kann aber nach Bedarf auch auf dem Schaft verschoben werden.

Der Sensor kann in einer bevorzugten Ausführung in der Spitzenregion 3 des Katheters 1 streng seitlich, also parallel zur Katheterlängsachse platziert werden, so dass bei einer Druckmessung der rein statische Druck erfasst wird und die additive oder subtraktive Messung eines Staudruckanteils vermieden wird.

Um auch langfristig Gerinnselbildungen (Thrombenablagerungen) auf dem im Gefäß 4 befindlichen Katheterabschnitt zu vermeiden, wird bevorzugt eine antithrombogene Beschichtung (z.B. mit Heparin) auf dieses Kathetersegment aufgebracht. Eine zusätzliche Beschichtung mit Zytostatika kann ein Zellwachstum auf der intravasalen Katheter- oder Sensorfläche verhindern.

Mit derzeit verfügbaren Drucksensoren kann das System z.B. für ein Langzeit-Blutdruckmonitoring eingesetzt werden, z.B. für Bluthochdruck- und Herzinsuffizienz-Patienten. Sobald andere Sensoren verfügbar sind, können auch andere Erkrankungen überwacht werden: z.B. Blutzuckerspiegel bei Diabetes-Patienten oder Sauerstoff-/Kohlendioxid-Partialdruck bei Patienten mit chronischen Lungenerkrankungen (z.B. COPD).

## Patentansprüche

1. Vorrichtung zur Erfassung physiologischer Meßgrößen im Körperinneren umfassend einen Katheter mit einer sensortragenden Spitze (3), der in ein Blutgefäß einführbar ist, wobei der Katheter (1) mit einer Transpondereinheit (2) zur drahtlosen Übertragung wenigstens einer Meßgröße an eine äußere Lesestation verbunden ist, wobei die Transpondereinheit nach Einführung des Katheters (1) in ein Blutgefäß (4) außerhalb des Blutgefäßes (4) angeordnet ist, wobei Katheter (1) und Transponder (2) flüssigkeitsdicht versiegelt sind, **dadurch gekennzeichnet, dass** die Vorrichtung eine vormontierte, koaxial um den Katheterschaft (1) angeordnete Manschette (12) aufweist, die in einem dem Sensor zugewandten Ende einer aufreißbaren ersten Hülse (10) angeordnet ist und die Vorrichtung eine auf dem Katheterschaft vormontierte zweite Hülse (11) umfasst, die koaxial auf dem transpondernahen Abschnitt des Katheterschaftes (1) angeordnet ist und die als Gegenhalter für die Manschette (12) dient, wobei die erste Hülse (10) zum Freigeben der Manschette (12) gegen die fixierte im Durchmesser kleinere zweite Hülse (11) zurückbewegbar ist.

2. Vorrichtung nach Anspruch 1, **dadurch gekennzeichnet, dass** mit der zweiten Hülse als Notschleuse ein Gefäßzugang erhaltbar ist.

3. Vorrichtung nach Anspruch 1 oder 2, **dadurch gekennzeichnet, dass** die Manschette aus blutstillendem Material ist.

4. Vorrichtung nach einem der vorherigen Ansprüche, **dadurch gekennzeichnet dass** die Hülsen (10,11) wenigstens eine Längsschlitzung (7) wenigstens an ihrem äußeren, gefäßfernen Ende (6a) aufweisen und durch Aufreißen in Längsrichtung des Katheters (1) von diesem entfernbar sind.

5. Vorrichtung nach einem der vorherigen Ansprüche, **dadurch gekennzeichnet, dass** an der Katheterspitze (3) und/oder auf dem Katheterschaft (1) wenigstens eine Markierung (13) angeordnet ist, die als Orientierung hinsichtlich der im Gefäß implantierten Katheterlänge dient.

6. Vorrichtung nach Anspruch 5, **dadurch gekennzeichnet, dass** die wenigstens eine Markierung als röntgendichte Punkte, Ringe, längsverlaufende Fäden oder Knicke ausgebildet ist.

7. Vorrichtung nach einem der vorherigen Ansprüche, **dadurch gekennzeichnet, dass** ein sensomahes seitliches Loch (14a) im Katheter (1) über einen im Katheter (1) verlaufenden Kanal (15) mit einem zweiten transpondernahen seitlichen Loch (14b) im Katheter (1) verbunden ist zur Katheter-Positionskontrolle und/oder zur Kontrastmittelgabe während der Implantationsprozedur.

8. Vorrichtung nach einem der vorherigen Ansprüche, **dadurch gekennzeichnet dass** ein Fixationsclip (16) am Katheterschaft (1) angeordnet ist, der mit einem Faden und/oder einer Klammer fassbar ist und mittels dem das System am benachbarten Gewebe fixierbar ist.

9. Vorrichtung nach Anspruch 8, **dadurch gekennzeichnet, dass** der Fixationsclip (16) am Katheterschaft (1) verschiebbar ist.

10. Vorrichtung nach einem der vorherigen Ansprüche, **dadurch gekennzeichnet, dass** der Sensor seitlich am Katheter (1) angeordnet ist, wobei die Sensorfläche eine Orientierung parallel zur Katheterachse aufweist.

11. Vorrichtung nach einem der vorherigen Ansprüche, **dadurch gekennzeichnet, dass** wenigstens ein sich im Gefäß befindlicher Katheterabschnitt eine antithrombogene und/oder eine zytostatische Beschichtung aufweist.

## Claims

1. Device for detecting physiological measured variables within the body comprising a catheter with a point (3) carrying a sensor, which may be inserted into a blood vessel, in which the catheter (1) is connected to a transponder unit (2) for wireless transfer of at least one variable to an external reading station, in which after introducing the catheter (1) into a blood vessel (4) the transponder unit is arranged outside the blood vessel (4), in which the catheter (1) and the transponder (2) are sealed so that they are impervious to liquid, **characterised in that** the device has a pre-assembled sleeve (12), which is arranged coaxially around the catheter axis (1), which is arranged in one end of a first casing (10), which can be torn open, facing towards the sensor and the device comprises a pre-assembled second casing (11) on the catheter axis, which is arranged coaxially on the section of the catheter axis (1) near the transponder and which serves as a brace for the sleeve (12), in which the first casing (10) can be moved back against the fixed second casing (11), which is smaller in diameter, to release the sleeve (12).

2. Device according to claim 1, **characterised in that** access to a vessel can be obtained with the second casing as an emergency lock.

3. Device according to claim 1 or 2, **characterised in that** the sleeve is made of blood staunching material.

4. Device according to one of the previous claims, **characterised in that** the casings (10,11) have at least one longitudinal slit (7) at least on their external end (6a) away from the vessel and can be removed from it by tearing open the catheter (1) in the longitudinal direction.

5. Device according to one of the previous claims **characterised in that** at least one mark (13) is arranged on the catheter point (3) and/or on the catheter axis (1), which serves for orientation with reference to the length of catheter implanted in the vessel.

6. Device according to claim 5, **characterised in that** at least one mark is made as dots, rings, longitudinally running threads or creases, which are impervious to X rays.

7. Device according to one of the previous claims, **characterised in that** a lateral hole (14a) in the catheter (1) near the sensor is connected to a second lateral hole (14b) in the catheter (1) near the transponder through a channel (15) running in the catheter (1) to control the catheter position and/or supply a contrast medium during the implantation procedure.

8. Device according to one of the previous claims, **characterised in that** a fastening clip (16) is arranged on the catheter axis (1), which can be held with a thread and/or a staple and by means of which the system can be fastened to the adjacent tissue.

9. Device according to claim 8, **characterised in that** the fastening clip (16) can be moved on the catheter axis (1).

10. Device according to one of the previous claims, **characterised in that** the sensor is arranged laterally on the catheter (1), in which the sensor area has an orientation parallel to the catheter axis.

11. Device according to one of the previous claims, **characterised in that** at least one catheter section located in the vessel has an antithrombogenic and/or a cytostatic coating.

## Revendications

1. Dispositif pour l'acquisition de grandeurs de mesure physiologiques à l'intérieur d'un corps comportant un cathéter doté d'une pointe (3) portant le capteur et pouvant être introduit dans un vaisseau sanguin, le cathéter (1) étant relié à une unité transpondeur (2) destinée à transmettre sans fil au moins une grandeur de mesure à un poste de lecture extérieur, l'unité transpondeur, une fois le cathéter (1) introduit dans un vaisseau sanguin (4), étant disposée à l'extérieur du vaisseau sanguin (4), le cathéter (1) et le transpondeur (2) étant scellés d'une manière étanche au fluide, **caractérisé en ce que** le dispositif comprend une manchette (12) pré-assemblée, disposée de manière coaxiale autour de la tige de cathéter (1) et disposée dans une extrémité tournée vers le capteur d'une première douille (10) pouvant être ouverte par déchirement et le dispositif comporte une seconde douille (11) pré-assemblée sur la tige de cathéter, disposée coaxialement sur la section proche du transpondeur de la tige de cathéter (1) et servant de pièce de contre-appui pour la manchette (12), la première douille (10) pouvant être déplacée en arrière pour libérer la manchette (12) contre la seconde douille (11) fixée dont le diamètre est plus petit.

2. Dispositif selon la revendication 1, **caractérisé en ce qu'**un accès au vaisseau peut être obtenu avec la seconde douille comme sas de secours.

3. Dispositif selon la revendication 1 ou 2,
**caractérisé en ce que** la manchette est composée d'un matériau hémostatique,

4. Dispositif selon l'une quelconque des revendications précédentes, **caractérisé en ce que** les douilles (10, 11) comprennent au moins un rainurage longitudinal (7) au moins sur son extrémité extérieure (6a) éloignée du vaisseau et, du fait qu'elles peuvent être ouvertes par déchirement dans la direction longitudinale du cathéter (1), peuvent être retirées dudit cathéter.

5. Dispositif selon l'une quelconque des revendications précédentes, **caractérisé en ce qu'**au moins un marquage (13) servant d'orientation en ce qui concerne la longueur du cathéter implanté dans le vaisseau est disposé sur la pointe de cathéter (3) et / ou sur la tige de cathéter (1).

6. Dispositif selon la revendication 5, **caractérisé en ce que** le ou les marquages sont conçus comme des points radio-opaques, des anneaux, des fils s'étendant longitudinalement ou des courbures.

7. Dispositif selon l'une quelconque des revendications précédentes, **caractérisé en ce qu'**un trou latéral (14a) proche du capteur est relié dans le cathéter (1) par un canal (15) s'étendant dans le cathéter (1) à un second trou latéral (14b) proche du transpondeur dans le cathéter (1) pour contrôler la position du cathéter et / ou pour délivrer un produit de contraste pendant la procédure d'implantation.

8. Dispositif selon l'une quelconque des revendications précédentes, **caractérisé en ce qu'**un clip de fixation (16) pouvant être saisi avec un fil et / ou une pince et permettant de fixer le système sur le tissu adjacent est disposé sur la tige de cathéter (1).

9. Dispositif selon la revendication 8, **caractérisé en ce que** le clip de fixation (16) peut être déplacé sur la tige de cathéter (1).

10. Dispositif selon l'une quelconque des revendications précédentes, **caractérisé en ce que** le capteur est disposé sur le côté du cathéter (1), la surface du capteur comprenant une orientation parallèle à l'axe du cathéter.

11. Dispositif selon l'une quelconque des revendications précédentes, **caractérisé en ce qu'**au moins une section de cathéter située dans le vaisseau comprend un revêtement antithombogène et / ou cytostatique.
